(19) **Europäisches Patentamt**
**European Patent Office**
**Office européen des brevets**

(11) **EP 2 727 593 A1**

(12) **EUROPEAN PATENT APPLICATION**
published in accordance with Art. 153(4) EPC

(43) Date of publication:
**07.05.2014 Bulletin 2014/19**

(51) Int Cl.:
*A61K 31/455* (2006.01)   *A61K 9/19* (2006.01)
*A61K 47/10* (2006.01)   *A61K 47/12* (2006.01)
*A61P 9/04* (2006.01)   *A61P 9/10* (2006.01)

(21) Application number: **12805113.3**

(22) Date of filing: **29.06.2012**

(86) International application number:
**PCT/JP2012/066743**

(87) International publication number:
**WO 2013/002382 (03.01.2013 Gazette 2013/01)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.06.2011   JP 2011145547**

(71) Applicant: **Mochida Pharmaceutical Co., Ltd.**
**Tokyo 160-8515 (JP)**

(72) Inventors:
• **YURA Takayuki**
**Tokyo 160-8515 (JP)**

• **HASHIMOTO Yuki**
**Tokyo 160-8515 (JP)**
• **KANEHIRO Hideo**
**Tokyo 160-8515 (JP)**

(74) Representative: **Grünecker, Kinkeldey,**
**Stockmair & Schwanhäusser**
**Leopoldstrasse 4**
**80802 München (DE)**

(54) **NICORANDIL-CONTAINING PHARMACEUTICAL COMPOSITION**

(57)   Provided is a pharmaceutical composition comprising nicorandil, mannitol, and an alkali metal salt of an organic carboxylic acid, wherein (1) the ratio of "a value of an X-ray diffraction peak of $\delta$-form crystals of the mannitol at 9.8° $\pm$ 0.3° (2θ)"/"a value of an X-ray diffraction peak of nicorandil crystals at 10.6° $\pm$ 0.3° (2θ)" is 2.0 or less, or (2) the ratio of "an intensity of a peak of $\delta$-form crystals of the mannitol at 1.01 THz $\pm$ 0.2 THz in a second derivative spectrum of an absorption spectrum obtained by terahertz transmission spectroscopy"/"an intensity of a peak of nicorandil crystals at 0.95 THz $\pm$ 0.2 THz in the second derivative spectrum of the absorption spectrum obtained by the terahertz transmission spectroscopy" is 1.5 or less. This pharmaceutical composition is excellent in storage stability.

EP 2 727 593 A1

**Description**

Technical Field

The present invention relates to a nicorandil-containing pharmaceutical composition having excellent storage stability and a method for producing the pharmaceutical composition.

Background Art

In Japan, nicorandil (chemical name: N-(2-hydroxyethyl)nicotinamide nitrate) is clinically available as an agent for treating angina in the form of tablets for oral administration and also as an agent for treating unstable angina and acute heart failure in the form of freeze-dried preparations for intravenous administration. As in the case of other nitro coronary dilators, nicorandil is unstable in an aqueous solution. Hence, for intravenous administration, it is necessary to prepare a solution by dissolving a freeze-dried preparation in a predetermined solvent such as saline or 5% glucose injection before use.

A standard of a freeze-dried nicorandil preparation specifies that the measured amount of nicorandil prepared in the form of a solution should be 95.0 to 115.0% of the labeled amount, and the amount of nitrate ions determined by a purity test should be 0.8% or less (where the labeled amount of nicorandil is taken as 100%) (Non Patent Literature 1). It is known that, even when nicorandil is in the form of a freeze-dried preparation, the nicorandil content decreases and degradation products such as nitrate ions increase with the lapse of time during storage period. For a currently commercially available freeze-dried nicorandil preparation, 10°C or below is described in Storage Method/Storage Condition and 2 years is described in Shelf Life (Non Patent Literature 2).

For this reason, for example, the following problems may arise: cold storage facilities are necessary for storing the freeze-dried preparations in manufacturing plants, during distribution, and in hospitals; and since it is necessary to take out the freeze-dried preparation from the refrigerator just before use, it is difficult to prepare a solution of nicorandil in advance, and the risk of a medical error increases in an emergency.

Non Patent Literature 2 discloses a freeze-dried nicorandil preparation obtained by adding, to nicorandil, D-mannitol in an amount resulting in a D-mannitol/nicorandil mass ratio of 1.5 and an appropriate amount of sodium citrate hydrate, followed by freeze-drying. The decrease in nicorandil content of this preparation after storage at 25°C for 9 months is 0.9 to 3.0%; 10°C or below is described in Storage Method/Storage Condition and 2 years is described in Shelf Life; and storage of the preparation at room temperature (1 to 30°C) is prohibited.

Meanwhile, proposals are presented for improving the storage stability of a freeze-dried nicorandil preparation.

Patent Literature 1 discloses a non-solution-type injection in which an alkali metal salt of a specific carboxylic acid such as citric acid is used in combination with nicorandil (Claim 1). In Example of Patent Literature 1, a freeze-dried nicorandil preparation is disclosed to which D-mannitol is added in an amount resulting in a D-mannitol/nicorandil mass ratio of 15 and to which sodium citrate was added in an amount resulting in a sodium citrate/nicorandil mass ratio of 2.5. It is described that the residual ratio of nicorandil in the preparation after storage at 40°C for 30 days is 89.0%. As will be described later, it seems that storage conditions of 40°C and 30 days for a freeze-dried nicorandil preparation are equivalent to storage conditions of 25°C and about 1 year. Hence, the shelf life of this preparation stored at room temperature is presumably less than 1 year.

Patent Literature 2 discloses a method for producing a stable freeze-dried nicorandil preparation, comprising adjusting the pH of an aqueous nicorandil solution to 4 or below by using fumaric acid, followed by freeze-drying (Claim 1). In Examples of Patent Literature 2, a freeze-dried nicorandil preparation is disclosed which is prepared by adding, to nicorandil, D-mannitol in an amount resulting in a D-mannitol/nicorandil mass ratio of 5 and an appropriate amount of citric acid. It is described that the residual ratio of nicorandil in this preparation after storage at 40°C for 1 week is 78.4%, and, presumably, it is substantially impossible to distribute or store this preparation at room temperature.

Patent Literature 3 discloses a method for producing a stable nicorandil powder, comprising dissolving a specific stabilizer such as an basic amino acid in an aqueous nicorandil solution, followed by drying (Claim 1). In Example of Patent Literature 3, a freeze-dried nicorandil preparation is disclosed which is obtained by adding, to nicorandil, D-mannitol in an amount resulting in a D-mannitol/nicorandil mass ratio of 1.5, L-arginine in an amount resulting in a L-arginine/nicorandil mass ratio of 0.83, and sodium citrate in an amount resulting in a sodium citrate/nicorandil mass ratio of 0.33. It is described that the residual ratio of nicorandil in this preparation after storage at 50°C for 7 days is 89.5%. As will be described later, it seems that the storage conditions of 50°C and 7 days for a freeze-dried nicorandil preparation are equivalent to storage conditions of 25°C and about 1 year and several months. Hence, the shelf life of the preparation stored at room temperature is presumably less than 1 year.

As described above, no nicorandil preparation which can be stored at room temperature for 1 year or more, for example, for 2 years has been in practical use. Accordingly, there is a demand for a nicorandil preparation, for example, a freeze-dried preparation, having excellent storage stability at room temperature in the medical field.

Citation List

Patent Literatures

> Patent Literature 1: Japanese Examined Patent Application Publication No. Hei 7-13018
> Patent Literature 2: Japanese Examined Patent Application Publication No. Hei 5-69088
> Patent Literature 3: Japanese Patent Application Publication No. 2001-187735

Non Patent Literatures

> Non Patent Literature 1: Document concerning stability test of NICORANDIL INTRAVENOUS FOR DRIP USE 2 mg "F", 12 mg "F", and 48 mg "F", Fuji Pharma Co., Ltd., April, 2008
> Non Patent Literature 2: Drug Interview Form, Sigmart (Registered Trademark) Injection, Chugai Pharmaceutical Co., Ltd., June, 2010, 7th revised edition

Summary of Invention

An object of the present invention is to provide a nicorandil-containing pharmaceutical composition having excellent storage stability, and a method for producing the nicorandil-containing pharmaceutical composition, especially, to provide a nicorandil pharmaceutical composition, for example, a freeze-dried preparation, which is stable in long-term storage at room temperature, and a method for producing the nicorandil pharmaceutical composition.

The present inventors have conducted intensive studies to develop a nicorandil composition stable in long-term storage especially at room temperature. As a result, it has been astonishingly found that when the components disclosed in Patent Documents 1 to 3 are used, but conditions not specifically disclosed in Patent Documents 1 to 3 are employed, a nicorandil-containing pharmaceutical composition extremely excellent in long-term storage stability and an excellent method for producing the composition can be obtained. The present invention has been completed on the bases of this finding.

Specifically, the present invention provides a pharmaceutical composition comprising nicorandil, mannitol, and an alkali metal salt of an organic carboxylic acid, wherein

> (1) the ratio of "a value of an X-ray diffraction peak of δ-form crystals of the mannitol at 9.8° ± 0.3° (2θ) "/"a value of an X-ray diffraction peak of nicorandil crystals at 10.6° ± 0.3° (2θ)" is 2.0 or less, or
> (2) the ratio of "an intensity of a peak of δ-form crystals of the mannitol at 1.01 THz ± 0.2 THz in a second derivative spectrum of an absorption spectrum obtained by terahertz transmission spectroscopy"/"an intensity of a peak of nicorandil crystals at 0.95 THz ± 0.2 THz in the second derivative spectrum of the absorption spectrum obtained by the terahertz transmission spectroscopy" is 1.5 or less.

The present invention also provides the pharmaceutical composition comprising the nicorandil, the mannitol, and the alkali metal salt of the organic carboxylic acid, wherein the mass ratio of the mannitol/the nicorandil is 0.25 to 1.25.

The present invention also provides the pharmaceutical composition comprising the nicorandil, the mannitol, and the alkali metal salt of the organic carboxylic acid, wherein the molar ratio of the mannitol/the alkali metal salt of the organic carboxylic acid is 0.4 to 4.0.

The present invention also provides the pharmaceutical composition, which is in a form of a freeze-dried composition.

The present invention also provides the pharmaceutical composition, which is produced by a freeze-drying method comprising performing a heating treatment to -3°C to 3°C and preferably to -3 °C to 0°C at least once in a step of freeze-drying an aqueous solution containing the nicorandil, the mannitol, and the alkali metal salt of the organic carboxylic acid.

The present invention also provides a freeze-dried composition comprising nicorandil, mannitol, and an alkali metal salt of an organic carboxylic acid, wherein the freeze-dried composition is produced by a freeze-drying method comprising performing a heating treatment to -3°C to 3°C and preferably to -3°C to 0°C at least once in a step of freeze-drying an aqueous solution containing the nicorandil, the mannitol, and the alkali metal salt of the organic carboxylic acid.

The present invention also provides a method for producing a pharmaceutical composition, comprising freeze-drying an aqueous solution containing nicorandil, mannitol, and an alkali metal salt of an organic carboxylic acid, wherein

a heating treatment to -3°C to 3°C and preferably to -3°C to 0°C is performed at least once in the freeze-drying step.

The present invention also provides a method for stabilizing nicorandil in a pharmaceutical composition comprising nicorandil, mannitol, and an alkali metal salt of an organic carboxylic acid, comprising causing the nicorandil and the mannitol to be present with

(1) the ratio of "a value of an X-ray diffraction peak of δ-form crystals of the mannitol at 9.8° ± 0.3° (2θ) "/"a value of an X-ray diffraction peak of nicorandil crystals at 10.6° ± 0.3° (2θ) " of the pharmaceutical composition being 2.0 or less, or

(2) the ratio of "an intensity of a peak of δ-form crystals of the mannitol at 1.01 THz ± 0.2 THz in a second derivative spectrum of an absorption spectrum obtained by terahertz transmission spectroscopy"/"an intensity of a peak of nicorandil crystals at 0.95 THz ± 0.2 THz in the second derivative spectrum of the absorption spectrum obtained by the terahertz transmission spectroscopy" of the pharmaceutical composition being 1.5 or less.

The present invention also provides a method for estimating storage stability of a pharmaceutical composition comprising nicorandil, mannitol, and an alkali metal salt of an organic carboxylic acid, the method comprising measuring the pharmaceutical composition for determining

(1) whether the ratio of "a value of an X-ray diffraction peak of δ-form crystals of the mannitol at 9.8° ± 0.3° (2θ) "/"a value of an X-ray diffraction peak of nicorandil crystals at 10.6° ± 0.3° (2θ)" is 2.0 or less, or

(2) whether the ratio of "an intensity of a peak of δ-form crystals of the mannitol at 1.01 THz ± 0.2 THz in a second derivative spectrum of an absorption spectrum obtained by terahertz transmission spectroscopy"/"an intensity of a peak of nicorandil crystals at 0.95 THz ± 0.2 THz in the second derivative spectrum of the absorption spectrum obtained by the terahertz transmission spectroscopy" is 1.5 or less.

The present invention also provides a method for estimating storage stability of a pharmaceutical composition comprising nicorandil, mannitol, and an alkali metal salt of an organic carboxylic acid, wherein the pharmaceutical composition is estimated to be stable at room temperature for 2 years, if the ratio of "a value of an X-ray diffraction peak of δ-form crystals of the mannitol at 9.8° ± 0.3° (2θ) "/"a value of an X-ray diffraction peak of nicorandil crystals at 10.6° ± 0.3° (2θ)" is 2.0 or less.

The present invention also provides a method for estimating storage stability of a pharmaceutical composition comprising nicorandil, mannitol, and an alkali metal salt of an organic carboxylic acid, wherein the pharmaceutical composition is estimated to be stable at room temperature for two years, if the ratio of "an intensity of a peak of δ-form crystals of the mannitol at 1.01 THz ± 0.2 THz in a second derivative spectrum of an absorption spectrum obtained by terahertz transmission spectroscopy"/"an intensity of a peak of nicorandil crystals at 0.95 THz ± 0.2 THz in the second derivative spectrum of the absorption spectrum obtained by the terahertz transmission spectroscopy" is 1.5 or less.

According to the present invention, the ratio of δ-form crystals of mannitol/nicorandil crystals in the nicorandil composition is low. Hence, a nicorandil composition, especially, a freeze-dried nicorandil composition, which is excellent in storage stability even at room temperature, can be obtained. Specifically, the nicorandil composition excellent in storage stability even at room temperature can be obtained by adding, to nicorandil, mannitol and one or more alkali metal salts of organic carboxylic acids at a specific ratio and/or by providing a heating treatment (also referred to as annealing) step performed under a specific condition in the freeze-drying step. A particularly preferable pharmaceutical composition of the present invention achieves an excellent effect of being stable at room temperature for 2 years. Hence, the pharmaceutical composition can be stored at room temperature in manufacturing plants, during distribution, and in hospitals, and is advantageous in that the need for cold storage facilities is eliminated. This enables an automatic dispenser to be used in a hospital, the pharmaceutical composition to be set on a tray or the like together with other preparations on the day before use, and the pharmaceutical composition to be set in advance on a medicine shelf or a tray for emergency outpatient services in preparation for emergency use, and hence the risk of a medical error is expected to decrease. The composition of the present invention, especially, the freeze-dried composition, has at least one of and preferably all of the above-described preferable effects. The method for estimating storage stability of a pharmaceutical composition of the present invention enables the estimation of the storage stability immediately after the freeze-drying, and makes it possible to specify a pharmaceutical composition stable at room temperature for 2 years without actually conducting a long-term storage test or an acceleration test.

Brief Description of Drawings

Fig. 1 is an X-ray diffraction chart of δ-form crystals of D-mannitol in Test Example 2.

Fig. 2 is an X-ray diffraction chart of nicorandil in Test Example 2.

Fig. 3 is a graph showing the correlation between the mixing ratio of δ-form crystals of D-mannitol/nicorandil and the intensity ratio (9.8°/10.6°) of the intensity of a peak at around 9.8° and the intensity of a peak at around 10.6° in X-ray diffraction charts in Test Example 2.

Fig. 4 is an X-ray diffraction chart of Preparation Example 1 in Test Example 2.

Fig. 5 is an X-ray diffraction chart of Preparation Example 6 in Test Example 2.

Fig. 6 is an X-ray diffraction chart of Preparation Example 13 in Test Example 2.

Fig. 7 is an X-ray diffraction chart of Preparation Example 16 in Test Example 2.

Fig. 8 is an X-ray diffraction chart of Reference Preparation 1 in Test Example 2.

Fig. 9 is an X-ray diffraction chart of Reference Preparation 2 in Test Example 2.

Fig. 10 is an absorption spectrum of δ-form crystals of D-mannitol obtained by terahertz transmission spectroscopy in Test Example 3.

Fig. 11 is an absorption spectrum of nicorandil obtained by terahertz transmission spectroscopy in Test Example 3.

Fig. 12 is a second derivative spectrum of the absorption spectrum of δ-form crystals of D-mannitol obtained by terahertz transmission spectroscopy in Test Example 3.

Fig. 13 is a second derivative spectrum of the absorption spectrum of nicorandil obtained by terahertz transmission spectroscopy in Test Example 3.

Fig. 14 is a graph showing the correlation between the mixing ratio of δ-form crystals of D-mannitol/nicorandil and the intensity ratio (1.01 THz/0.95 THz) of the intensity of a peak at around 1.01 THz and the intensity of a peak at around 0.95 THz in second derivative spectra of absorption spectra obtained by terahertz transmission spectroscopy in Test Example 3.

Fig. 15 is a second derivative spectrum of an absorption spectrum of Preparation Example 1 obtained by terahertz transmission spectroscopy in Test Example 3.

Fig. 16 is a second derivative spectrum of an absorption spectrum of Preparation Example 13 obtained by terahertz transmission spectroscopy in Test Example 3.

Fig. 17 is a second derivative spectrum of an absorption spectrum of Preparation Example 33 obtained by terahertz transmission spectroscopy in Test Example 3.

Fig. 18 is a second derivative spectrum of an absorption spectrum of Preparation Example 16 obtained by terahertz transmission spectroscopy in Test Example 3.

Fig. 19 is a second derivative spectrum of an absorption spectrum of Reference Preparation 1 obtained by terahertz transmission spectroscopy in Test Example 3.

Description of Embodiments

A pharmaceutical composition to which the present invention is directed contains nicorandil, mannitol, and an alkali metal salt of an organic carboxylic acid as essential components.

As the nicorandil, nicorandil specified under the Japanese Pharmacopoeia can be readily available.

As the mannitol, D-mannitol is preferably used. Note that mannitol in various crystal forms can be used, and it is preferable not to use δ-form crystals.

The organic carboxylic acid is preferably citric acid, tartaric acid, acetic acid, malic acid, succinic acid, or the like, and particularly preferably citric acid. The alkali metal salt includes potassium salts, sodium salts, and the like, and is preferably a sodium salt. Of these salts, trisodium citrate (referred to simply as sodium citrate in some case) and disodium citrate are particularly preferable.

In the present invention, the mass ratio of the mannitol/ the nicorandil used is preferably 0.25 to 1.25 and more preferably 0.25 to 1.0. In addition to or independently from this ratio, the molar ratio of the mannitol/the alkali metal salt of the organic carboxylic acid used is preferably 0.4 to 4.0 (the mass ratio is 0.25 to 2.5 in the case of sodium citrate) and more preferably 1.2 to 3.2 (the mass ratio is 0.75 to 2.0 in the case of sodium citrate).

In the present invention, trometamol (tris(hydroxymethyl)aminomethane) is preferably added in addition to the above-described essential components. The amount of trometamol added may be any, and trometamol is preferably used with the mass ratio of the trometamol/the nicorandil being 0.1 to 1.5 and more preferably 0.1 to 0.5.

In the present invention, additives conventionally added to a pharmaceutical composition, such as an excipient, a surfactant, a coloring agent, and a sweetener, can be further added as additional components. Here, the excipient includes saccharides such as lactose, white soft sugar, and glucose; starches such as pregelatinized starch and dextrin; celluloses such as crystalline cellulose, microcrystalline cellulose, and hydroxypropyl cellulose; magnesium aluminometasilicate; and silicon dioxide. The surfactant includes sodium lauryl sulfate, sucrose fatty acid esters, and the like. The sweetener includes sucrose, aspartame, and the like. Moreover, a lubricant such as magnesium stearate or talc can be added.

The pharmaceutical composition of the present invention is preferably produced by a method for producing a pharmaceutical composition, comprising freeze-drying an aqueous solution containing nicorandil, mannitol, and an alkali metal salt of an organic carboxylic acid, wherein a heating treatment to -3°C to 3°C and preferably to -3°C to 0°C is performed at least once in the freeze-drying step.

Here, the aqueous solution containing nicorandil, mannitol, and an alkali metal salt of an organic carboxylic acid is preferably obtained by dissolving these components in water, preferably distilled water for injection, so that the mass ratio of the mannitol/the nicorandil and/or the molar ratio of the mannitol/the alkali metal salt of the organic carboxylic acid can be within the above-described ranges. Here, the concentration of mannitol is preferably adjusted to 1.25 to 10% by mass and further preferably 1.25 to 8% by mass. Likewise, when trometamol is used, it is preferable to dissolve trometamol in water, so that the mass ratio of the trometamol/ the nicorandil can be within the above-described range.

The pH of the thus prepared aqueous solution is preferably adjusted to a range from substantially neutral to weakly alkaline and more preferably adjusted to 7 to 9.

The thus prepared aqueous solution is subjected to freeze-drying including sequentially a step of preliminary freezing, a step of heating treatment (annealing), and a drying step, in a usual manner. After the heating treatment (annealing), a secondary freezing step may be conducted. The drying step may be divided into two steps constituted of a primary (sublimation) step and a secondary (dehydration) step, or into three or more steps. For example, it is preferable that the aqueous solution be frozen by performing the preliminary freezing at -20°C to -60°C, preferably at -30°C to -50°C, and more preferably at -35°C to -45°C, and then the heating treatment (annealing) be performed to -3°C to 3°C and preferably -3°C to 0°C at least once. It is more preferable to perform the heating treatment (annealing) to -2°C to 0°C, and further preferable to perform the heating treatment (annealing) to -1°C to 0°C. A heating treatment (annealing) to 0°C is particularly preferable. The temperature 0°C is about a temperature at which the frozen material obtained by freezing the aqueous solution does not completely turn into an aqueous solution. The heating treatment is preferably performed for 20 minutes to 21 days, more preferably 30 minutes to 7 days, and further preferably 40 minutes to 72 hours, in total. In particular, the heating treatment (annealing) time only needs to be 40 minutes or more for a 2 mg nicorandil preparation, 2 hours or more for a 12 mg preparation, and 8 hours or more for a 48 mg preparation.

Regarding the secondary freezing temperature, the secondary freezing can be conducted in the same manner as the preliminary freezing. The primary (sublimation) step in the drying step can be performed under ordinary reduced pressure at a temperature of -10°C to -40°C and preferably -15°C to -30°C for 2 hours to 7 days and preferably 5 to 72 hours. The secondary (dehydration) step in the drying step can be performed under ordinary reduced pressure at a temperature of 10°C to 60°C and preferably 30°C to 50°C for 3 hours to 72 hours and preferably 6 to 24 hours.

The above-described additional components which have been added to the aqueous solution together with the essential components can be subjected to the freeze-drying treatment. However, it is preferable to mix the additional components after the freeze-drying of the essential components.

The ratio of "a value of an X-ray diffraction peak of δ-form crystals of the mannitol at around 9.8° (2θ)"/"a value of an X-ray diffraction peak of nicorandil crystals at around 10.6° (2θ)" of the pharmaceutical composition of the present invention is preferably 2.0 or less. Here, the angles (2θ) of the X-ray diffraction peaks may be shifted by about ±0.3°, ±0.2°, or ±0.1° (mainly, translational shift), among measurements. For this reason, in this description, the angle around 9.8° (2θ) is preferably 9.8° ± 0.3° (2θ), 9.8° ± 0.2° (2θ), or 9.8° ± 0.1° (2θ), and the angle around 10.6° (2θ) is preferably 10.6° ± 0.3° (2θ), 10.6° ± 0.2° (2θ), or 10.6° ± 0.1° (2θ).

In the present invention, for example, a pharmaceutical composition having the above-described perk ratio can be easily obtained by the above-described freeze-drying. Here, the ratio of "a value of an X-ray diffraction peak of δ-form crystals of the mannitol at 9.8° ± 0.3° (2θ) "/"a value of an X-ray diffraction peak of nicorandil crystals at 10.6° ± 0.3° (2θ)" is preferably 0 to 2.0 and more preferably 0 to 1.0.

The ratio of "a value of an X-ray diffraction peak of δ-form crystals of the mannitol at 9.8° ± 0.3° (2θ)" and "the value of an X-ray diffraction peak of nicorandil crystals at 10.6° ± 0.3° (2θ)," can be easily calculated, for example, as follows. Specifically, an X-ray diffraction chart is obtained by using an X-ray diffractometer (Miniflex, Rigaku Corporation, or D8 DISCOVER with GADDS, Bruker AXS) in a usual manner using Cu-Kα radiation. Then, the value of a peak at around 9.8° (2θ) and the value of a peak at around 10.6° (2θ) in the chart are actually measured, and the ratio between these values is calculated. Here, the X-ray diffraction measurement is conducted preferably within 1 hour and more preferably within 30 minutes after the preparation of the pharmaceutical composition.

The ratio of "an intensity of a peak of δ-form crystals of the mannitol at around 1.01 THz in a second derivative spectrum of an absorption spectrum obtained by terahertz transmission spectroscopy"/"an intensity of a peak of nicorandil crystals at around 0.95 THz in the second derivative spectrum of the absorption spectrum obtained by the terahertz transmission spectroscopy" of the pharmaceutical composition of the present invention is preferably 1.5 or less. Here, the second derivative spectrum of an absorption spectrum obtained by terahertz transmission spectroscopy may be shifted by about ±0.2 THz or ±0.1 THz (mainly, translational shift), among measurements. For this reason, in this description, the frequency at around 1.01 THz is preferably 1.01 THz ± 0.2 THz or 1.01 THz ± 0.1 THz, and the frequency at around 0.95 THz is preferably 0.95 THz ± 0.2 THz or 0.95 THz ± 0.1 THz.

In the present invention, for example, a pharmaceutical composition having the peak intensity ratio can be easily obtained by the above-described freeze-drying. Here, the ratio of "an intensity of a peak of δ-form crystals of the mannitol at 1.01 THz ± 0.2 THz in a second derivative spectrum of an absorption spectrum obtained by terahertz transmission spectroscopy"/"an intensity of a peak of nicorandil crystals at 0.95 THz ± 0.2 THz in the second derivative spectrum of the absorption spectrum obtained by the terahertz transmission spectroscopy" of the pharmaceutical composition may be 1.5 or less and preferably 1.3 or less.

Regarding the second derivative spectrum of an absorption spectrum of the δ-form crystals of the mannitol and the nicorandil crystals obtained by terahertz transmission spectroscopy, the second derivative spectrum and the peak intensities can be obtained, for example, as follows. Specifically, an absorption spectrum is obtained by transmission spectroscopic measurement using a terahertz spectroscopy system (TAS7500, ADVANTEST CORPORATION) with a frequency resolution of 7.6 GHz and a number of scans of 8192. Then, a second derivative spectrum is obtained from the obtained absorption spectrum, and the peak intensities are obtained. After that, the ratio of the obtained peak intensities is calculated. Here, the terahertz transmission spectroscopy is conducted preferably within 1 hour and more preferably within 30 minutes after preparation of the pharmaceutical composition.

Furthermore, in the present invention, nicorandil in a pharmaceutical composition comprising nicorandil, mannitol, and an alkali metal salt of an organic carboxylic acid can be stabilized by causing the nicorandil and the mannitol to be present with (1) the ratio of "a value of an X-ray diffraction peak of δ-form crystals of the mannitol at 9.8° ± 0.3° (2θ) "/"a value of an X-ray diffraction peak of nicorandil crystals at 10.6° ± 0.3° (2θ)" of the pharmaceutical composition being 2.0 or less and preferably 1.0 or less, or (2) the ratio of "an intensity of a peak of δ-form crystals of the mannitol at 1.01 THz ± 0.2 THz in a second derivative spectrum of an absorption spectrum obtained by terahertz transmission spectroscopy"/"an intensity of a peak of nicorandil crystals at 0.95 THz ± 0.2 THz in the second derivative spectrum of the absorption spectrum obtained by the terahertz transmission spectroscopy" of the pharmaceutical composition being 1. 5 or less and preferably 1. 3 or less. In this stabilization method, the ratio of the values of the peaks and the like are the same as described above.

Moreover, in the present invention, the following method for estimating storage stability of a pharmaceutical composition comprising nicorandil, mannitol, and an alkali metal salt of an organic carboxylic acid can be used. Specifically, the estimation method comprises measuring the pharmaceutical composition for determining

(1) whether the ratio of "a value of an X-ray diffraction peak of δ-form crystals of the mannitol at 9.8° ± 0.3° (2θ) "/"a value of an X-ray diffraction peak of nicorandil crystals at 10.6° ± 0.3° (2θ)" is 2.0 or less and preferably 1.0 or less, or (2) whether the ratio of "an intensity of a peak of δ-form crystals of the mannitol at 1.01 THz ± 0.2 THz in a second derivative spectrum of an absorption spectrum obtained by terahertz transmission spectroscopy"/"an intensity of a peak of nicorandil crystals at 0.95 THz ± 0.2 THz in the second derivative spectrum of the absorption spectrum obtained by the terahertz transmission spectroscopy" is 1.5 or less and preferably 1.3 or less. In this method for estimating storage stability, the ratio of the values of the peaks and the like are the same as described above.

Furthermore, in the present invention, the following method for estimating storage stability of a pharmaceutical composition comprising nicorandil, mannitol, and an alkali metal salt of an organic carboxylic acid can be used. Specifically, in the estimation method, a pharmaceutical composition is estimated to be stable at room temperature for 2 years, if the ratio of "a value of an X-ray diffraction peak of δ-form crystals of the mannitol at 9.8° ± 0.3° (2θ) "/"a value of an X-ray diffraction peak of nicorandil crystals at 10.6° ± 0.3° (2θ)" of the pharmaceutical composition is 2.0 or less and preferably 1.0 or less. In this method for estimating storage stability, the ratio of the values of the peaks and the like are the same as described above.

Furthermore, in the present invention, the following method for estimating storage stability of a pharmaceutical composition comprising nicorandil, mannitol, and an alkali metal salt of an organic carboxylic acid can be used. Specifically, in the estimation method, a pharmaceutical composition is estimated to be stable at room temperature for 2 years, if the ratio of "an intensity of a peak of δ-form crystals of the mannitol at 1.01 THz ± 0.2 THz in a second derivative spectrum of an absorption spectrum obtained by terahertz transmission spectroscopy"/"an intensity of a peak of nicorandil crystals at 0.95 THz ± 0.2 THz in the second derivative spectrum of the absorption spectrum obtained by the terahertz transmission spectroscopy" of the pharmaceutical composition is 1.5 or less and preferably 1.3 or less. In this method for estimating storage stability, the ratio of the values of the peaks and the like are the same as described above.

[Examples]

Next, the present invention is described more specifically by way of Examples; however, the present invention is not limited to these examples.

Preparation Example 1

300 mg of nicorandil (Japanese Pharmacopoeia grade), 75 mg of D-mannitol (product name: Mannite S, Mitsubishi Shoji Foodtech Co. Ltd.), and 300 mg of sodium citrate hydrate (Wako Pure Chemical Industries, Ltd.) were each weighed and dissolved in distilled water for injection. The pH was adjusted to the range from 7. 0 to 8.5 by adding citric acid (Wako Pure Chemical Industries, Ltd.) and/or sodium hydroxide (Wako Pure Chemical Industries, Ltd.) on an as-needed basis, and the total volume was adjusted to 50 mL. The solution was filtered through a 0.22-$\mu$m membrane filter, and then dispensed into glass vials having a body diameter of 33.0 mm (8 mL each). By using a freeze-drying apparatus (product name: Triomaster II-A04, Kyowa Vacuum Engineering. Co., Ltd.), the material was preliminarily frozen at -40°C and then freeze-dried. In a cooling step at the initial stage of the freeze-drying step, a heating treatment (annealing) with the material temperature being 0°C was conducted for 6 hours twice. The frozen material was not melted during the heating treatment step based on visual observation. After that, the material was dried under reduced pressure. In the freeze-drying, the other procedures were conducted in a usual manner. Then, the vials were each tightly sealed with a rubber stopper and an aluminum cap. Thus, a freeze-dried preparation was obtained.

Preparation Examples 2, 6, 7, 11, 13, 14

Freeze-dried preparations were obtained according to the formulations shown in Table 1 (showing the content of each component per glass vial) by the same production method as that of Preparation Example 1.

Preparation Example 3

300 mg of nicorandil (Japanese Pharmacopoeia grade), 75 mg of D-mannitol (product name: Mannite S, Mitsubishi Shoji Foodtech Co. Ltd.), and 300 mg of sodium citrate hydrate (Wako Pure Chemical Industries, Ltd.) were weighed and dissolved in distilled water for injection. The pH was adjusted to the range from 7.0 to 8.5 by adding citric acid (Wako Pure Chemical Industries, Ltd.) and/or sodium hydroxide (Wako Pure Chemical Industries, Ltd.) on an as-needed basis, and the total volume was adjusted to 50 mL. The solution was filtered through a 0.22-$\mu$m membrane filter, and then dispensed into glass vials having a body diameter of 24.5 mm (2 mL each). By using a freeze-drying apparatus (product name: Triomaster II-A04, Kyowa Vacuum Engineering. Co., Ltd.), the material was preliminarily frozen at -40°C and freeze-dried. In a cooling step at the initial stage of the freeze-drying step, a heating treatment (annealing) with the material temperature being 0°C was conducted for 8 hours twice. The frozen material was not melted during the heating treatment step based on visual observation. After that, the material was dried under reduced pressure. In the freeze-drying, the other procedures were conducted in a usual manner. Then, the vials were each tightly sealed with a rubber stopper and an aluminum cap. Thus, a freeze-dried preparation was obtained.

Preparation Examples 4, 5, 8 to 10, 12, and 15 to 17

Freeze-dried preparations were obtained according to the formulations shown in Table 1 (showing the content of each component per glass vial) by the same production method as that of Preparation Example 3.

Table 1 shows the formulations (showing the content of each component per glass vial) of Preparation Examples 1 to 17.

Table 1

| Preparation Example | Nicorandil (mg) | D-mannitol (mg) | Sodium citrate (mg) |
|---|---|---|---|
| 1 | 48 | 12 | 48 |
| 2 | 48 | 12 | 8 |
| 3 | 12 | 4.5 | 12 |
| 4 | 12 | 4.5 | 6 |
| 5 | 12 | 4.5 | 3 |
| 6 | 48 | 18 | 7.2 |
| 7 | 48 | 36 | 144 |
| 8 | 12 | 9 | 12 |
| 9 | 12 | 9 | 6 |

(continued)

| Preparation Example | Nicorandil (mg) | D-mannitol (mg) | Sodium citrate (mg) |
|---|---|---|---|
| 10 | 12 | 9 | 3 |
| 11 | 48 | 48 | 32 |
| 12 | 12 | 12 | 6 |
| 13 | 48 | 60 | 40 |
| 14 | 48 | 60 | 24 |
| 15 | 12 | 18 | 12 |
| 16 | 12 | 18 | 6 |
| 17 | 12 | 18 | 3 |

Preparation Example 18

A freeze-dried preparation was obtained by freeze-drying conducted by the same method as the production method of Preparation Example 9, except that 3 mg of trometamol (tris(hydroxymethyl)aminomethane, Wako Pure Chemical Industries, Ltd.) was further added to the formulation of Preparation Example 9, and that two heating treatments (annealing) in total were conducted in the freeze-drying at 0°C for 3 hours and at -4°C for 24 hours, respectively.

Preparation Examples 19 to 28-5

By the same production method as that in Preparation Example 1, solutions were dispensed according to the formulations shown in Table 2 (showing the content of each component per glass vial) into glass vials (the 2 mg preparation was poured into glass vials having a body diameter of 18.1 mm, the 12 mg preparations were poured into glass vials having a body diameter of 24.5 mm, the 48 mg preparations of Preparation Examples 19 to 27 and 29 to 36 were poured into glass vials having a body diameter of 33.0 mm, and the 48 mg preparations of Preparation Examples 28 to 28-5 were poured into glass vials having a body diameter of 30.0 mm). These glass vials were subjected to freeze-drying in the same production method as that in Preparation Example 1, except that the heating treatment (annealing) in the freeze-drying was conducted under the conditions shown in Table 3. Thus, freeze-dried preparations were obtained. In Preparation Example 28-3, partial melting of the frozen material and precipitation of nicorandil were observed during the heating treatment step under visual observation. In Preparation Example 28-4, the frozen material was partially melted until 1.6 hours had passed during the heating treatment step, and completely melted at the end of the heating treatment step. Hence, the production was discontinued.

Table 2

| Formulation | Nicorandil (mg) | D-mannitol (mg) | Sodium citrate (mg) | Trometamol (mg) | Distilled water for injection (mL) |
|---|---|---|---|---|---|
| A | 12 | 18 | 6 | - | 2 mL |
| B | 12 | 9 | 6 | - | 2 mL |
| C | 2 | 1.5 | 1 | 0.5 | 0.4 mL |
| D | 48 | 36 | 24 | - | 6 mL |
| E | 48 | 36 | 24 | 12 | 6 mL |

Table 3

| Preparation Example | Formulation | Heating treatment step (temperature·duration×number of times) |
|---|---|---|
| 19 | A | None |
| 20 | A | -10°C·3hr×1 |
| 21 | A | -4°C·3hr×1, -4°C·24hr×1 |

(continued)

| Preparation Example | Formulation | Heating treatment step (temperature·duration×number of times) |
|---|---|---|
| 22 | A | 0°C·3hr×1, -4°C·24hr×1 |
| 23 | B | 0°C·6hr×2 |
| 24 | B | 0°C·3hr×1, -4°C·24hr×1 |
| 25 | B | 0°C·2hr×2 |
| 26 | B | 0°C·1hr×2 |
| 27 | C | 0°C·20min×2 |
| 28 | D | 0°C·6hr×2 |
| 28-1 | D | 0°C·6hr×1 |
| 28-2 | D | 0°C·24hr×2 |
| 28-3 | D | 1°C·3hr×2 |
| 28-4 | D | 3°C·12hr×1 |
| 28-5 | E | 0°C·4hr×2 |

Preparation Example 29 to 38

Freeze-dried preparations were obtained by the same production method as that in Preparation Example 28, except that sodium citrate in Preparation Example 28 was replaced as shown in the formulations in Table 4 (showing the content of each component per glass vial). The compounds listed in Table 4 were all obtained from Wako Pure Chemical Industries, Ltd.

Table 4

| Preparation Example | Compound used in place of sodium citrate | Formulated amount (mg) |
|---|---|---|
| 29 | Sodium L-tartrate | 18.8 |
| 30 | Sodium L-tartrate | 75.1 |
| 31 | Sodium acetate | 6.7 |
| 32 | Sodium acetate | 26.8 |
| 33 | Disodium succinate | 22.0 |
| 34 | Disodium succinate | 88.2 |
| 35 | Sodium hydrogen phosphate hydrate | 29.2 |
| 36 | Sodium hydrogen carbonate | 27.4 |

Reference Preparation 1

A freeze-dried preparation (product name: Sigmart (Registered Trademark) 12 mg injection, Chugai Pharmaceutical Co., Ltd.) containing 12 mg of nicorandil, 18 mg of D-mannitol, and an appropriate amount of sodium citrate hydrate was stored at approximately 4°C, and taken out before use.

Reference Preparation 2

A freeze-dried preparation (product name: NICORANDIL intravenous for drip use 12 mg "F", Fuji Pharma Co., Ltd.) containing 12 mg of nicorandil, 18 mg of D-mannitol, and 6 mg of sodium citrate hydrate was stored at approximately 4°C, and taken out before use.

Test Example 1 <Storage Stability Test>

The freeze-dried preparations were stored in thermostatic chambers at 25°C and 60% relative humidity (RH), at 40°C and 75% RH, and/or at 50°C and 30% RH. The preparations were sampled over time and measured for the amount of nicorandil and/or the amount of nitrate ions, which are a degradation product of nicorandil, by the following methods.

Nicorandil quantification method:

Each of the preparations was dissolved in a mobile phase solution shown below to obtain a sample solution having a nicorandil concentration of 1 mg/mL, which was calculated by using the amount of nicorandil formulated in the preparation. For example, the nicorandil 12 mg preparation of Preparation Example 3 was dissolved in 12 mL of the mobile phase solution. Separately, 20 mg of nicorandil was weighed, and the volume was adjusted exactly to 20 mL by adding the mobile phase solution to the nicorandil to obtain a standard solution. Under the conditions shown below, the sample solutions and the standard solution (10 $\mu$L each) were measured by liquid chromatography to determine the peak areas $A_T$ and $A_S$ of nicorandil.

Measurement conditions

Column: a stainless steel tube having an inner diameter of 4 mm and a length of 25 cm and filled with octadecylsilylated silica gel for liquid chromatography having a particle diameter of 5 $\mu$m.
Column temperature: a constant temperature at around 25°C.
Mobile phase solution: a mixture liquid of water/tetrahydrofuran/triethylamine/trifluoroacetic acid (982:10:5:3).

Measurement wavelength: 254 nm

The amount of nicorandil in each preparation was calculated by the following formula (I).

$$\text{Formula (I): the amount (mg) of nicorandil} = \text{the amount of nicorandil formulated} \times A_T / A_S$$

The amount of nicorandil at the start of the test was taken as 100%, and the residual ratio (%) of nicorandil in each preparation after the storage was calculated according to the following formula (II).

$$\text{Formula (II): the residual ratio (\%) of nicorandil} = \text{the amount of nicorandil after the storage/the amount of nicorandil at the start of the test} \times 100$$

Nitrate ion quantification method:

Each preparation was dissolved in a mobile phase solution to obtain a sample solution having a nicorandil concentration of 1 mg/mL or 1.2 mg/mL, which was calculated by using the amount of nicorandil formulated to the preparation. Separately, 27.4 mg and 82.2 mg (20 mg and 60 mg in terms of nitrate ions) of sodium nitrate were weighed, and the volumes were adjusted exactly to 20 mL and 50 mL by adding the mobile phase solution. The solutions were further diluted 125-fold with the mobile phase solution to obtain standard solutions. By using 10 $\mu$L of each of the sample solutions and the standard solutions, the peak areas $A_T$ and $A_S$ of nitrate ions were measured under the same conditions as those of the nicorandil quantification method, except that the measurement wavelength was changed to 210 nm.
The amount of nicorandil formulated in each preparation was taken as 100%, and the amount (%) of nitrate ions in the preparation was calculated according to the following formula (III).

$$\text{Formula (III): the amount (\%) of nitrate ions} = A_T / A_S \times 100 / 125$$

Tables 5 to 7 show the results of the calculation of the residual ratio (%) of nicorandil and/or the amount (%) of nitrate ions.

Table 5

| Preparation Example | Amount (%) of nitrate ions | |
|---|---|---|
| | 25°C, 60%RH, 12 months | 50°C, 30%RH, 6 days |
| 1 | - | 0.37 |
| 2 | - | 0.38 |
| 3 | 0.55 | - |
| 4 | 0.51 | - |
| 5 | 0.39 | - |
| 6 | - | 0.45 |
| 7 | - | 0.43 |
| 8 | 0.45 | - |
| 9 | 0.41 | 0.53 |
| 11 | - | 0.38 |
| 12 | 0.55 | - |
| 13 | - | 0.37 |
| 14 | - | 0.50 |
| 16 | 0.85 | - |
| 17 | 0.87 | - |
| Reference Preparation 1 | 0.87 | 0.84 |

In this table, Preparation Examples 1 to 14 correspond to products of the present invention, and Preparation Examples 16 and 17 are Comparative Examples.

In each of Preparation Example 16 and 17 and Reference Preparation 1, the amount of nitrate ions after the storage at 25°C and 60%RH for 12 months was 0.8% or higher. Since the value of the amount of nitrate ions specified under the standard of a freeze-dried nicorandil preparation is 0.8% or less, it is difficult to store these preparations at room temperature for 12 months. On the other hand, in each of Preparation Examples 3 to 5, 8 to 9, and 12, the amount of nitrate ions after the storage at 25°C and 60%RH for 12 months was 0.39 to 0.55%. Hence, these preparations can be stored at room temperature for at least 12 months. In each of Preparation Examples 1, 2, 6, 7, 11, 13, and 14, the amount of nitrate ions after the storage at 50°C and 30%RH for 6 days was 0.37 to 0.50%, which infers that these preparations can be stored at 25°C for approximately 1 year. Moreover, presumably, the preparations of Preparation Examples 1 to 14 can be stored at room temperature for longer periods, for example, for 18 months or 24 months or more, and it has been found that some of these preparations can be stored at 25°C and 60%RH for 24 months or more.

This is presumably because differences arose in crystallization of the components, especially, D-mannitol during the freeze-drying because of the following reason. Specifically, the D-mannitol/nicorandil mass ratio was 1.5 in Preparation Examples 16 and 17 and Reference Preparation 1, whereas the mass ratio took a low value of 0.25 to 1.25 in Preparation Examples 1 to 14. In addition, the D-mannitol/sodium citrate mass ratio was 3 to 6 in Preparation Examples 16 and 17, whereas the mass ratio took a low value of 0.25 to 2.5 in Preparation Examples 1 to 9 and 11 to 14. The low D-mannitol/nicorandil mass ratio and/or the low D-mannitol/sodium mass ratio led to the differences in crystallization of the components, especially, D-mannitol during the freeze-drying, as will be described later.

Table 6

| Preparation Example | Amount of nitrate ions (%) | | | |
|---|---|---|---|---|
| | 40°C, 75%RH | | 50°C, 30%RH | |
| | 4 weeks | 8 weeks | 6 days | 13 days |
| 9 | - | - | 0.53 | 1.11 |
| 24 | 0.38 | 0.51 | 0.44 | 0.73 |
| 18 | 0.31 | 0.39 | 0.40 | 0.58 |

The preparation of Preparation Example 18 had a smaller amount of nitrate ions under all the storage conditions than those of Preparation Examples 9 and 24. This was presumably because the buffering effect of trometamol reduced the decrease in pH due to nitrate ions generated with the decomposition of nicorandil, so that further promotion of the decomposition of nicorandil due to the decrease in pH was suppressed.

In the case of a freeze-dried nicorandil preparation, the storage conditions at 40°C and 75%RH for 4 weeks and 8 weeks seem to be equivalent to storage conditions at 25°C for approximately 1 year and approximately 2 years, and the storage conditions at 50°C and 30%RH for 6 days and 13 days seem to be equivalent to storage conditions at 25°C for approximately 1 year and approximately 3 years. Hence, presumably, these preparations can be stored at room temperature for 2 years or more.

Table 7

| Preparation Example | Amount of nitrate ions (%) 50°C, 30%RH, 6 days | Residual ratio of nicorandil (%) 50°C, 30%RH, 12 days |
|---|---|---|
| 19 | - | 35.9 |
| 20 | - | 44.2 |
| 21 | 1.02 | - |
| 22 | 0.58 | - |
| 23 | 0.53 | 97.6 |
| 24 | 0.44 | - |
| 25 | 0.40 | - |
| 26 | 0.43 | - |
| 27 | 0.41 | - |
| 28 | 0.41 | - |
| 28-1 | 0.57 | - |
| 28-2 | 0.38 | - |
| 28-3 | 0.34 | - |
| 28-5 | 0.32 | - |
| Reference Preparation 1 | 0.84 | 91.1 |

In the table, Preparation Examples 19 to 21 are Comparative Examples, and Preparation Examples 22 to 28-5 correspond to products of the present invention.

The residual ratios of nicorandil of Preparation Examples 19 and 20 after the storage at 50°C and 30%RH for 12 days took low values of 35.9% and 44.2%, respectively. The amount of nitrate ions of Preparation Example 21 after the storage at 50°C and 30%RH for 6 days took a high value of 1.02%. On the other hand, among Preparation Examples 22 to 28-5, the residual ratio of nicorandil after the storage at 50°C and 30%RH for 12 days took a high value of 97.6% and/or the amounts of nitrate ions after the storage at 50°C and 30%RH for 6 days took low values of 0.32 to 0.58%.

The was presumably because the conditions of Preparation Example 19 where no heating treatment (annealing) was conducted during the freeze-drying and the conditions of Preparation Examples 20 and 21 where the heating treatment (annealing) was conducted at -10°C or -4°C were unsuitable as crystallization conditions for achieving storage stability. On the other hand, since the heating treatment (annealing) was conducted at 0 to 1°C in Preparation Examples 22 to 28-5, the heating treatment (annealing) presumably exerted a favorable effect on the crystallization for achieving storage stability during the freeze-drying.

In the case of a freeze-dried nicorandil preparation, the storage conditions of 50°C, 30%RH, and 6 days or 12 days seem to be equivalent to storage conditions of 25°C and approximately 1 year or about 2 years to 3 years. In addition, it seems that the time of the heating treatment (annealing) at 0°C only needs to be 40 minutes or more for 2 mg preparations, 2 hours or more for 12 mg preparations, and 8 hours or more for 48 mg preparations, and that the heating treatment (annealing) may be conducted at once or separately two times or more.

During the heating treatment step, it is desirable that not all the frozen material be melt. The time of the heating treatment (annealing) at 1°C is 9 hours or less, preferably 6 hours or less, and more preferably 3 hours or less for 48 mg preparations, and the time of the heating treatment (annealing) at 3°C is 1.6 hours or less, preferably 0.8 hours or less, and more preferably 0.4 hours or less for 48 mg preparations.

Table 8

| Preparation Example | Amount of nitrate ions (%) | |
|---|---|---|
| | Immediately after freeze-drying | 50°C, 30%RH, 6 days |
| 28 | 0.2 or less | 0.41 |
| 29 | 0.2 or less | 0.51 |
| 30 | 0.2 or less | 0.36 |
| 31 | 0.2 or less | 0.42 |
| 32 | 0.2 or less | 0.69 |
| 33 | 0.2 or less | 0.46 |
| 34 | 0.2 or less | 0.56 |
| 35 | 0.68 | 3.25 |
| 36 | 0.2 or less | 2.66 |

In this table, Preparation Examples 28 to 34 correspond to products of the present invention, and Preparation Examples 35 and 36 are Comparative Examples.

In Preparation Examples 29 to 34 where sodium citrate was replaced with sodium L-tartrate, sodium acetate, or disodium succinate, the amounts of nitrate ions were 0.2% or less immediately after the freeze-drying and 0.36 to 0.69% after the storage at 50°C and 30%RH for 6 days. These amounts of nitrate ions were not higher than 0.8%, which is the value of the amount of nitrate ions specified under the standard of a freeze-dried nicorandil preparation. Hence, presumably, these preparations can be stored at room temperature for 12 months or more. In addition, although the amounts of nitrate ions after the storage at 50°C and 30%RH for 6 days of Preparation Examples 29, 31, and 33 in each of which a sodium salt of an organic carboxylic acid was added in an amount equimolar to sodium citrate of Preparation Example 28 were slightly higher than that of Preparation Example 28, it seems that these sodium salts also have an effect of improving the storage stability of nicorandil as in the case of sodium citrate.

On the other hand, the amount of nitrate ions of Preparation Example 35 where sodium citrate was replaced with sodium hydrogen phosphate hydrate took a high value of 0.68% immediately after the freeze-drying and was 3.25% after the storage at 50°C and 30%RH for 6 days. In Preparation Example 36 where sodium citrate was replaced with sodium hydrogen carbonate, the amount of nitrate ions immediately after the freeze-drying was 0.2% or less, but the amount of nitrate ions after the storage at 50°C and 30%RH for 6 days took a high value of 2.66%.

Hence, it is impossible to store, at room temperature for 12 months or more, a preparation to which an alkali metal salt of an inorganic acid is added. In addition, it seems that sodium hydrogen phosphate hydrate does not have the effect of stabilizing nicorandil in the freeze-drying step.

Test Example 2 <X-ray diffraction measurement of freeze-dried compositions>

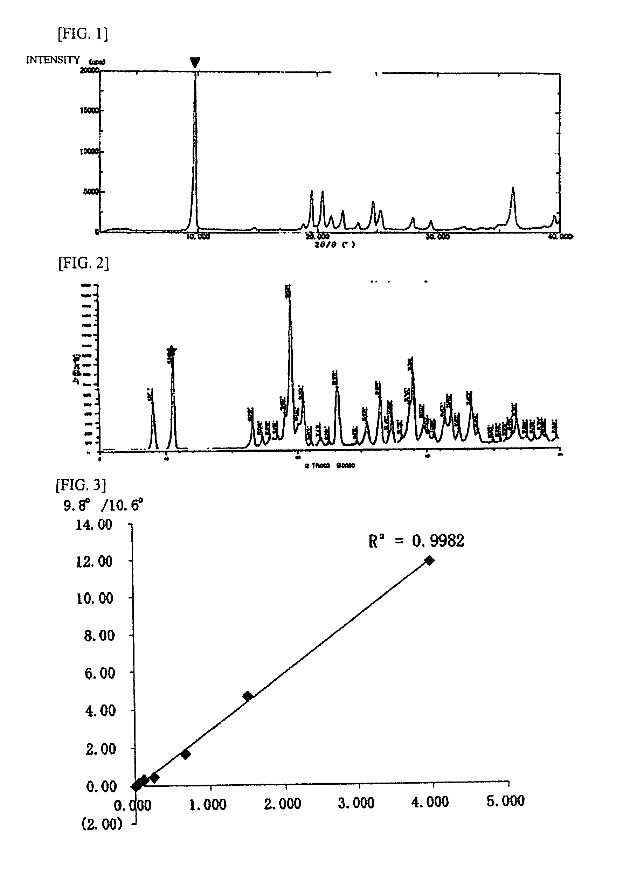

X-ray diffraction measurement of δ-form crystals of D-mannitol (product name: Parteck Delta M, Merck KGaA), nicorandil (Japanese Pharmacopoeia grade), and samples prepared by mixing them at the mass ratios shown in Table 9 was conducted by using an X-ray diffractometer (Miniflex, Rigaku Corporation or D8 DISCOVER with GADDS, Bruker AXS) in a usual manner. The measurement was started within 30 minutes after mixing each sample. Thus, X-ray diffraction charts and the peak intensities of the X-ray diffraction peaks were obtained. Note that peak intensities of peaks not higher than the detection limit of the X-ray diffractometer were regarded as 0. Fig. 1 shows the X-ray diffraction chart of the δ-form crystals of D-mannitol, and Fig. 2 shows the X-ray diffraction chart of nicorandil. The ratio between the intensity of the peak at around 9.8° (indicated by V in the chart), which is an X-ray diffraction reflection specific to δ-form crystals of D-mannitol, and the intensity of the peak at around 10.6° (indicated by ★ in the chart), which is an X-ray diffraction reflection specific to nicorandil, was calculated, and the correlation between the ratio of the intensities and the mixing ratio was obtained. Table 9 shows the intensity of the peak at around 9.8°, the intensity of the peak at around 10.6°, and the intensity ratio (9.8°/10.6°) therebetween. Fig. 3 shows a graph showing the correlation between the mixing ratio of δ-form crystals of D-mannitol/nicorandil and the 9.8°/10.6° ratio.

Table 9

| Mass ratio of δ-form crystals of D-mannitol/nicorandil | X-ray diffraction reflection peak intensities of each preparation | | Intensity ratio 9.8°/10.6° |
|---|---|---|---|
| | Around 9.8° | Around 10.6° | |
| 0.000 | 0 | 940 | 0.00 |
| 0.053 | 210 | 1460 | 0.14 |
| 0.111 | 470 | 1433 | 0.33 |
| 0.250 | 646 | 1445 | 0.45 |
| 0.667 | 2331 | 1382 | 1.69 |
| 1.500 | 3175 | 677 | 4.69 |
| 4.000 | 5777 | 488 | 11.84 |

In the range of the mass ratio of δ-form crystals of D-mannitol/nicorandil of from 0.000 to 4.000, the correlation coefficient ($R^2$) between the mass ratio and the 9.8°/10.6° ratio was 0.9982, and the obtained correlation was substantially linear as shown in Fig. 3.

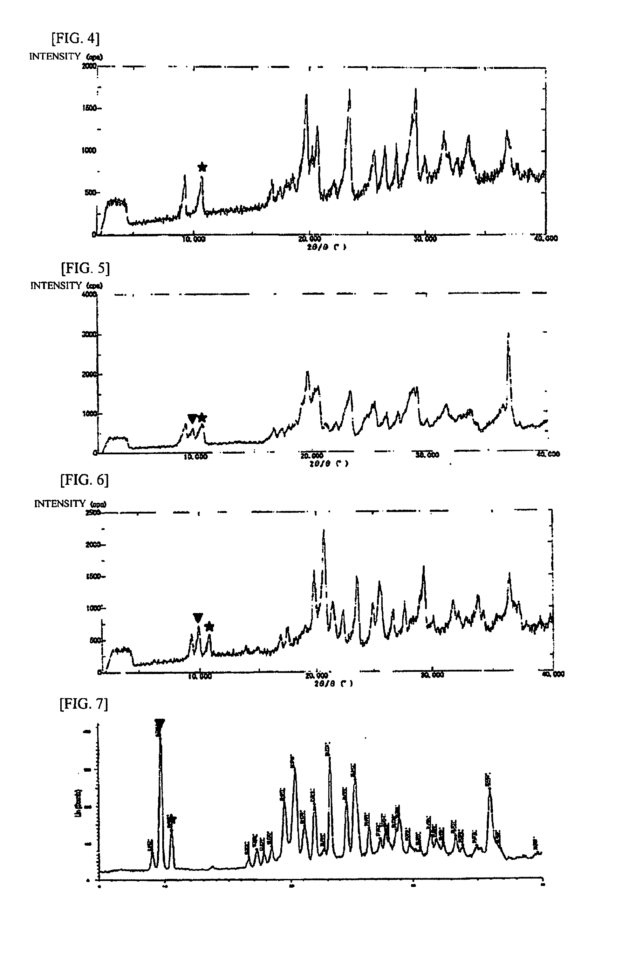

Preparation Examples and Reference Preparations shown in Table 10 were unsealed, and the freeze-dried compositions therein were powdered to prepare samples. X-ray diffraction measurement was started within 30 minutes after the unsealing in the same manner to obtain X-ray diffraction charts and the peak intensities of X-ray diffraction peaks. In addition, an X-ray diffraction chart of β-form crystals of D-mannitol (product name: Mannite S, Mitsubishi Shoji Foodtech Co. Ltd.), and the peak intensities of X-ray diffraction peaks thereof were obtained in the same manner. Figs. 4 to 9 show the X-ray diffraction charts of representative samples.

In the X-ray diffraction patterns of all Preparation Examples and Reference Preparations, the peaks at around 9.0° and 10.6°, which are X-ray diffraction reflections specific to nicorandil crystals, were observed. In the X-ray diffraction patterns of Preparation Examples 6, 13, 29, 31, 33, 16, and 35 and Reference Preparations 1 and 2, the peak at around 9.8°, which is an X-ray diffraction reflection specific to δ-form crystals of D-mannitol, was observed. In contrast, in the X-ray diffraction patterns of Preparation Examples 1, 2, 7, 9, 11, 28-2, and 28-3, the peak at around 9.8° was not observed. In addition, in any one of the X-ray diffraction patterns of Preparation Examples and Reference Preparations, the peak at around 29.5°, which is an X-ray diffraction reflection specific to β-form crystals of D-mannitol, was not observed. Hence, it seems that nicorandil crystals were present in all of the compositions of Preparation Examples and Reference Preparations, whereas almost no β-form crystals of D-mannitol were present therein. It seems that δ-form crystals of D-mannitol were present in the compositions of Preparation Examples 6, 13, 29, 31, 33, 16, and 35 and Reference Preparations 1 and 2. In contrast, it seems that no δ-form crystals of D-mannitol were present in the compositions of Preparation Examples 1, 2, 7, 9, 11, 28-2, and 28-3.

The intensity of the peak at around 9.8°, which is an X-ray diffraction reflection specific to δ-form crystals of D-mannitol, the intensity of the peak at around 10.6°, which is an X-ray diffraction reflection specific to nicorandil, and the 9.8°/10.6° ratio were calculated. Table 10 shows the calculation result of the 9.8°/10.6° ratio of each preparation.

Table 10

| Preparation Example | X-ray diffraction reflection peak intensities of each preparation | | Intensity ratio 9.8°/10.6° |
|---|---|---|---|
| | Around 9.8° | Around 10.6° | |
| 1 | 0 | 127 | 0 |
| 2 | 0 | 189 | 0 |

(continued)

| Preparation Example | X-ray diffraction reflection peak intensities of each preparation | | Intensity ratio 9.8°/10.6° |
|---|---|---|---|
| | Around 9.8° | Around 10.6° | |
| 6 | 61 | 133 | 0.46 |
| 7 | 0 | 91 | 0 |
| 9 | 0 | 69 | 0 |
| 11 | 0 | 108 | 0 |
| 13 | 141 | 86 | 1.64 |
| 28-2 | 0 | 163 | 0 |
| 28-3 | 0 | 50 | 0 |
| 29 | 217 | 160 | 1.36 |
| 31 | 177 | 186 | 0.95 |
| 33 | 128 | 223 | 0.57 |
| 16 | 377 | 131 | 2.88 |
| 35 | 301 | 128 | 2.35 |
| Reference Preparation 1 | 503 | 92.1 | 5.46 |
| Reference Preparation 2 | 528 | 136 | 3.88 |

In this table, Preparation Examples 1 to 13 and Preparation Examples 28-2 to 33 correspond to products of the present invention, and Preparation Example 16 and Preparation Example 35 are Comparative Examples.

Among the preparations to which an alkali metal salt of an organic carboxylic acid was added, the 9.8°/10.6° ratios of Preparation Example 16 and Reference Preparations 1 and 2 took high values of 2.88, 5.46 and 3.88, respectively. On the other hand, the ratios of Preparation Examples 1 to 13 and 28-2 to 33 took low values of 0 to 1.64, which indicated that the Preparation Examples 1 to 13 and 28-2 to 33 were in the state where the ratio of δ-form crystals of D-mannitol to nicorandil crystals was low. These results, in combination with the results of the storage stability test of Test Example 1, indicate that a preparation to which an alkali metal salt of an organic carboxylic acid was added and which has a 9.8°/10.6° ratio of 2.88 or higher is difficult to store at room temperature for 12 months. On the other hand, a preparation having a 9.8°/10.6° ratio of 1.64 or less can be stored at room temperature for at least 12 months. In addition, this preparation presumably can be stored for a longer period, for example, at room temperature for 18 months or 24 months or more. It has been found that some of such preparations can be stored at 25°C and 60%RH for 24 months or more.

Here, presumably, the formation of δ-form crystals of D-mannitol during the freeze-drying is suppressed because of the low D-mannitol/nicorandil mass ratio in the formulation of the freeze-dried composition, the low mass ratio of D-mannitol/the alkali metal salt of the organic carboxylic acid in the freeze-dried composition, and the freeze-drying step including the step of heating treatment, so that the storage stability of nicorandil is improved.

Test Example 3 <terahertz transmission spectroscopy of freeze-dried compositions>

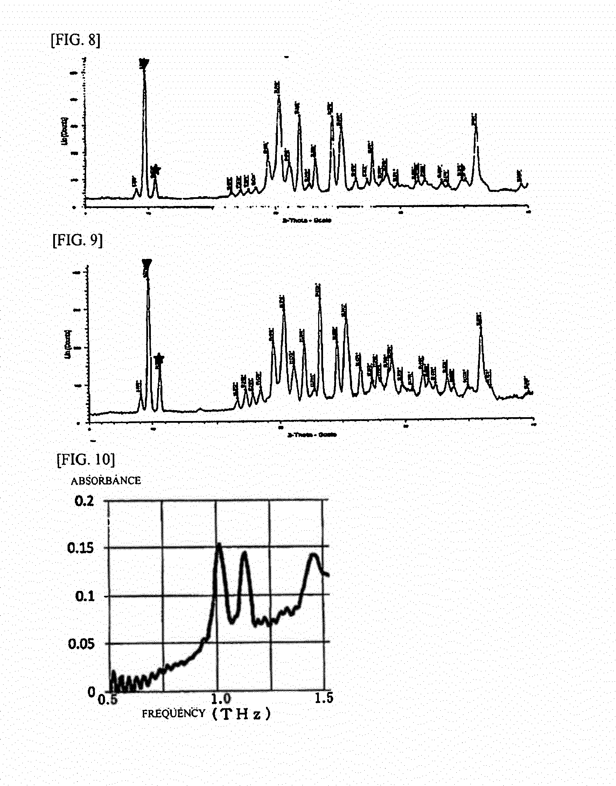

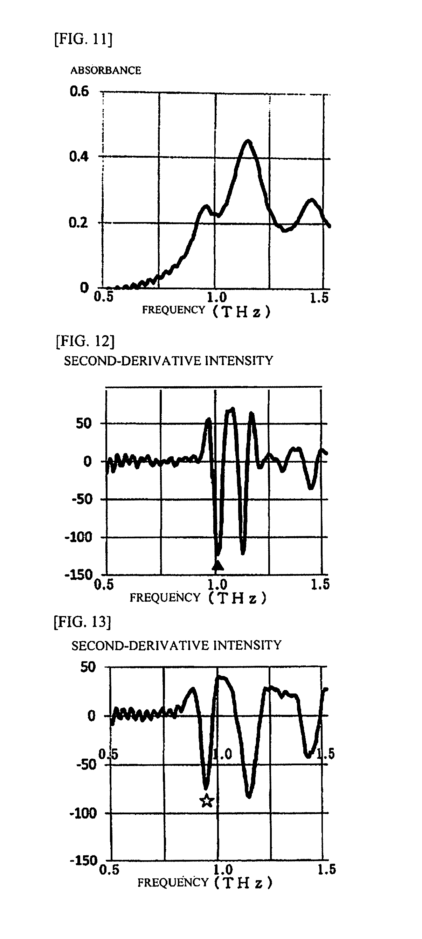

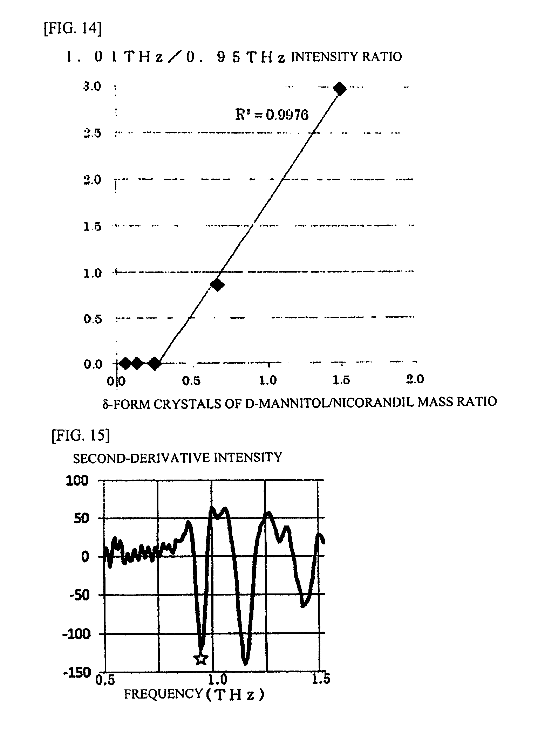

In an agate mortar, 70 mg each of δ-form crystals of D-mannitol, nicorandil (Japanese Pharmacopoeia grade), and samples obtained by mixing them at the mass ratios shown in Table 11 was mixed with 130 mg of polyethylene to homogeneity, and the mixture was compression-molded by using a pellet forming apparatus and a manual hydraulic press (STJ-10P, ST Japan INC.) at 14.5 kN under evacuation using a vacuum pump. Thus, a pellet having a diameter of 10 mm and a thickness of approximately 2.7 mm was prepared. Within 30 minutes after the start of the mixing, transmission spectroscopic measurement was conducted by using a terahertz spectroscopy system (TAS7500, ADVANTEST CORPORATION) at a frequency resolution of 7.6 GHz and with a number of scans of 8192 to obtain an absorption spectrum. From the obtained absorption spectrum, a second derivative spectrum and peak intensities were obtained. Note that the intensity of a peak not higher than the detection limit of the terahertz spectroscopy system was regarded as 0. Fig. 10 shows the absorption spectrum of the δ-form crystals of D-mannitol, and Fig. 11 shows the absorption spectrum of nicorandil. Fig. 12 shows the second derivative spectrum of the absorption spectrum of the δ-form crystals of D-mannitol, and Fig. 13 shows the second derivative spectrum of the absorption spectrum of nicorandil. The ratio of the peak intensity of the second derivative peak (indicated by A in the spectrum) at around 1.01 THz, which is specific to δ-form crystals of D-mannitol, to the peak intensity of the second derivative peak (indicated by ☆ in the spectrum) at around 0.95 THz, which is specific to nicorandil, was calculated, and the correlation between the intensity ratio and the mixing ratio was obtained. Table 11 shows the intensity ratio (1.01 THz/0.95 THz) of the intensity of the peak at around 1.01 THz to the intensity of the peak at around 0.95 THz, and Fig. 14 shows a graph showing the correlation between the mixing ratio of δ-form crystals of D-mannitol/nicorandil and the 1.01 THz/0.95 THz ratio.

Table 11

| Mass ratio of δ-form crystals of D-mannitol/nicorandil | Intensity ratio 1.01 THz/0.95 THz |
|---|---|
| 0.053 | 0 |
| 0.111 | 0 |
| 0.250 | 0 |
| 0.667 | 0.860 |
| 1.500 | 2.979 |

When the mass ratio of δ-form crystals of D-mannitol/nicorandil was 0.053 to 0.250, the peaks were lower than the detection limit, and the peak intensities were regarded as 0. In the range from 0.250 to 1.500, the correlation coefficient ($R^2$) between the mass ratio and the 1.01 THz/0.95 THz ratio was 0.9976, and the obtained correlation was substantially linear as shown in Fig. 14.

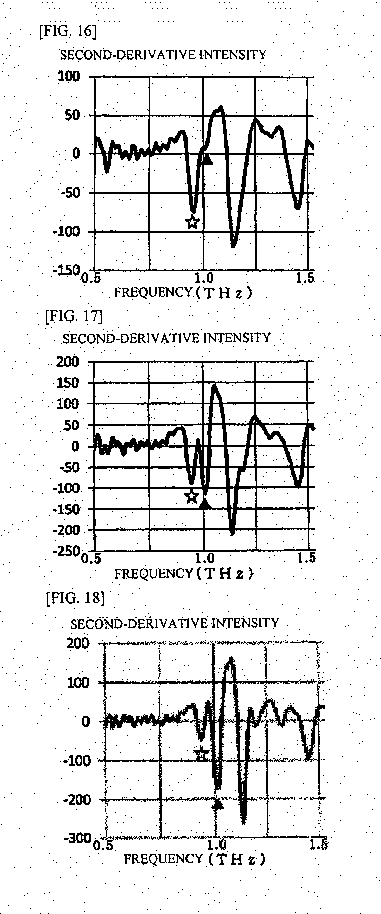

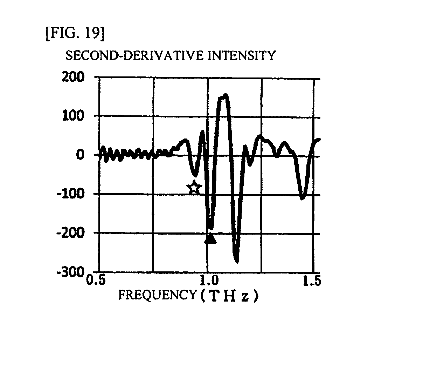

Preparation Examples and Reference Preparations shown in Table 12 were unsealed, and the freeze-dried compositions therein were powdered to prepare samples. Terahertz transmission spectroscopy was started within 30 minutes after the unsealing in the same manner to obtain absorption spectra, second derivative spectra of the absorption spectra, and the peak intensities of second derivative peaks. In addition, an absorption spectrum of β-form crystals of D-mannitol and a second derivative spectrum of the absorption spectrum were obtained by terahertz transmission spectroscopy in the same manner. Figs. 15 to 19 show the second derivative spectra of the absorption spectra of representative samples obtained by the terahertz transmission spectroscopy.

In all the second derivative spectrum patterns of Preparation Examples and Reference Preparations, the peak at around 0.95 THz, which is an absorption specific to nicorandil crystals, was observed. In the second derivative spectrum patterns of Preparation Examples 9, 13, 29, 31, 33, 16, and 35 and Reference Preparations 1 and 2, the peak at around 1.01 THz, which is an absorption specific to δ-form crystals of D-mannitol, was observed. In contrast, in the second derivative spectrum patterns of Preparation Examples 1, 2, 6, 7, and 11, the peak at around 0.95 THz was not observed. In addition, in all the second derivative spectrum patterns of Preparation Examples and Reference Preparations, the peak at around 1.50 THz, which is an absorption specific to δ-form crystals of D-mannitol, was not observed. Hence, it seems that nicorandil crystals were present in all the compositions of Preparation Examples and Reference Preparation, whereas almost no β-form crystals of D-mannitol were present therein. It seems that δ-form crystals of D-mannitol were present in the compositions of Preparation Examples 9, 13, 29, 31, 33, 16, and 35 and Reference Preparations 1 and 2, whereas no δ-form crystals of D-mannitol were present in the compositions of Preparation Examples 1, 2, 6, 7, and 11.

The ratio (1.01 THz/0. 95 THz) of the intensity of the peak at around 1.01 THz, which is an absorption specific to δ-form crystals of D-mannitol, and the intensity of the peak at around 0.95 THz, which is an absorption specific to nicorandil, was calculated. Table 12 shows the calculation result of the 1.01 THz/0.95 THz ratio of each preparation.

Table 12

| Preparation Example | Intensity ratio 1.01 THz/0.95 THz |
|---|---|
| 1 | 0 |
| 2 | 0 |
| 6 | 0 |
| 7 | 0 |
| 9 | 0.09 |
| 11 | 0 |
| 13 | 0.032 |
| 29 | 0.591 |

(continued)

| Preparation Example | Intensity ratio 1.01 THz/0.95 THz |
| --- | --- |
| 31 | 0.479 |
| 33 | 1.296 |
| 16 | 3.574 |
| 35 | 1.758 |
| Reference Preparation 1 | 3.643 |
| Reference Preparation 2 | 3.806 |

Among the preparations to which an alkali metal salt of an organic carboxylic acid was added, the 1.01 THz/0.95 THz ratios of Preparation Example 16 and Reference Preparations 1 and 2 took high values of 3.574, 3.643, and 3.806, respectively. On the other hand, the 1.01 THz/0.95 THz ratios of Preparation Examples 1 to 13 and 29 to 33 took low values of 0 to 1.296, which indicated that the preparations of Preparation Examples 1 to 13 and 29 to 33 were in the state where the ratio of $\delta$-form crystals of D-mannitol to nicorandil crystals was low. These results, in combination with the results of the storage stability test of Test Example 1, indicate that a preparation to which an alkali metal salt of an organic carboxylic acid is added and which has a 1.01 THz/0. 95 THz ratio of 3.574 or higher is difficult to store at room temperature for 12 months. On the other hand, a preparation having a 1.01 THz/0.95 THz ratio of 1.296 or lower can be stored at room temperature for at least 12 months. In addition, this preparation presumably can be stored for a longer period, for example, for 18 months or 24 months or more at room temperature. It has been found that some of such preparations can be stored at 25°C and 60%RH for 24 months or more.

Here, presumably, the formation of the $\delta$-form crystals of D-mannitol during the freeze-drying is suppressed because of the low D-mannitol/nicorandil mass ratio in the formulation of the freeze-dried composition, the low mass ratio of the D-mannitol/the alkali metal salt of the organic carboxylic acid in the freeze-dried composition, and the freeze-drying step including the step of heating treatment, so that the storage stability of nicorandil is improved.

**Claims**

1. A pharmaceutical composition comprising nicorandil, mannitol, and an alkali metal salt of an organic carboxylic acid, wherein

   (1) the ratio of "a value of an X-ray diffraction peak of $\delta$-form crystals of the mannitol at 9.8° $\pm$ 0.3° (2$\theta$) "/"a value of an X-ray diffraction peak of nicorandil crystals at 10.6° $\pm$ 0.3° (2$\theta$)" is 2.0 or less, or
   (2) the ratio of "an intensity of a peak of $\delta$-form crystals of the mannitol at 1.01 THz $\pm$ 0.2 THz in a second derivative spectrum of an absorption spectrum obtained by terahertz transmission spectroscopy"/"an intensity of a peak of nicorandil crystals at 0.95 THz $\pm$ 0.2 THz in the second derivative spectrum of the absorption spectrum obtained by the terahertz transmission spectroscopy" is 1.5 or less.

2. The pharmaceutical composition comprising the nicorandil, the mannitol, and the alkali metal salt of the organic carboxylic acid according to claim 1, wherein
   the ratio of "a value of an X-ray diffraction peak of $\delta$-form crystals of the mannitol at 9.8° $\pm$ 0.3° (2$\theta$)"/"a value of an X-ray diffraction peak of nicorandil crystals at 10.6° $\pm$ 0.3° (2$\theta$)" is 2.0 or less.

3. The pharmaceutical composition comprising the nicorandil, the mannitol, and the alkali metal salt of the organic carboxylic acid according to claim 1, wherein
   the ratio of "an intensity of a peak of $\delta$-form crystals of the mannitol at 1.01 THz $\pm$ 0.2 THz in a second derivative spectrum of an absorption spectrum obtained by terahertz transmission spectroscopy"/"an intensity of a peak of nicorandil crystals at 0.95 THz $\pm$ 0.2 THz in the second derivative spectrum of the absorption spectrum obtained by the terahertz transmission spectroscopy" is 1.5 or less.

4. The pharmaceutical composition according to any one of claims 1 to 3, wherein
   the mass ratio of the mannitol/the nicorandil is 0.25 to 1.25.

5. The pharmaceutical composition according to any one of claims 1 to 4, wherein

the molar ratio of the mannitol/the alkali metal salt of the organic carboxylic acid is 0.4 to 4.0.

6. The pharmaceutical composition according to any one of claims 1 to 5, which is in a form of a freeze-dried composition.

7. The pharmaceutical composition according to claim 6, wherein
the freeze-dried composition is produced by a freeze-drying method comprising performing a heating treatment to -3°C to 3°C at least once in a step of freeze-drying an aqueous solution containing the nicorandil, the mannitol, and the alkali metal salt of the organic carboxylic acid.

8. The pharmaceutical composition according to claim 6, wherein
the freeze-dried composition is produced by a freeze-drying method comprising performing a heating treatment to -3°C to 0°C at least once in a step of freeze-drying an aqueous solution containing the nicorandil, the mannitol, and the alkali metal salt of the organic carboxylic acid.

9. A freeze-dried composition comprising nicorandil, mannitol, and an alkali metal salt of an organic carboxylic acid, wherein
the freeze-dried composition is produced by a freeze-drying method comprising performing a heating treatment to -3°C to 3°C at least once in a step of freeze-drying an aqueous solution containing the nicorandil, the mannitol, and the alkali metal salt of the organic carboxylic acid.

10. A freeze-dried composition comprising nicorandil, mannitol, and an alkali metal salt of an organic carboxylic acid, wherein
the freeze-dried composition is produced by a freeze-drying method comprising performing a heating treatment to -3°C to 0°C at least once in a step of freeze-drying an aqueous solution containing the nicorandil, the mannitol, and the alkali metal salt of the organic carboxylic acid.

11. A method for producing a pharmaceutical composition, comprising freeze-drying an aqueous solution containing nicorandil, mannitol, and an alkali metal salt of an organic carboxylic acid, wherein
a heating treatment to -3°C to 3°C is performed at least once in the freeze-drying step.

12. A method for producing a pharmaceutical composition, comprising freeze-drying an aqueous solution containing nicorandil, mannitol, and an alkali metal salt of an organic carboxylic acid, wherein
a heating treatment to -3°C to 0°C is performed at least once in the freeze-drying step.

13. A method for stabilizing nicorandil in a pharmaceutical composition comprising nicorandil, mannitol, and an alkali metal salt of an organic carboxylic acid, comprising causing the nicorandil and the mannitol to be present with

(1) the ratio of "a value of an X-ray diffraction peak of δ-form crystals of the mannitol at 9.8° ± 0.3° (2θ)"/"a value of an X-ray diffraction peak of nicorandil crystals at 10.6° ± 0.3° (2θ)" of the pharmaceutical composition being 2. 0 or less, or
(2) the ratio of "an intensity of a peak of δ-form crystals of the mannitol at 1.01 THz ± 0.2 THz in a second derivative spectrum of an absorption spectrum obtained by terahertz transmission spectroscopy"/"an intensity of a peak of nicorandil crystals at 0.95 THz ± 0.2 THz in the second derivative spectrum of the absorption spectrum obtained by the terahertz transmission spectroscopy" of the pharmaceutical composition being 1.5 or less.

14. The method for stabilizing nicorandil in a pharmaceutical composition comprising nicorandil, mannitol, and an alkali metal salt of an organic carboxylic acid according to claim 13, wherein
the nicorandil and the mannitol are caused to be present with the ratio of "a value of an X-ray diffraction peak of δ-form crystals of the mannitol at 9.8° ± 0.3° (2θ)"/"a value of an X-ray diffraction peak of nicorandil crystals at 10.6° ± 0.3° (2θ)" of the pharmaceutical composition being 2.0 or less.

15. The method for stabilizing nicorandil in a pharmaceutical composition comprising nicorandil, mannitol, and an alkali metal salt of an organic carboxylic acid according to claim 14, wherein
the nicorandil and the mannitol are caused to be present with the ratio of "an intensity of a peak of δ-form crystals of the mannitol at 1.01 THz ± 0.2 THz in a second derivative spectrum of an absorption spectrum obtained by terahertz transmission spectroscopy"/"an intensity of a peak of nicorandil crystals at 0.95 THz ± 0.2 THz in the second derivative spectrum of the absorption spectrum obtained by the terahertz transmission spectroscopy" of the pharmaceutical composition being 1.5 or less.

16. A method for estimating storage stability of a pharmaceutical composition comprising nicorandil, mannitol, and an alkali metal salt of an organic carboxylic acid, the method comprising measuring the pharmaceutical composition for determining

    (1) whether the ratio of "a value of an X-ray diffraction peak of δ-form crystals of the mannitol at 9.8° ± 0.3° (2θ) "/"a value of an X-ray diffraction peak of nicorandil crystals at 10.6° ± 0.3° (2θ)" is 2.0 or less, or
    (2) whether the ratio of "an intensity of a peak of δ-form crystals of the mannitol at 1.01 THz ± 0.2 THz in a second derivative spectrum of an absorption spectrum obtained by terahertz transmission spectroscopy"/"an intensity of a peak of nicorandil crystals at 0.95 THz ± 0.2 THz in the second derivative spectrum of the absorption spectrum obtained by the terahertz transmission spectroscopy" is 1.5 or less.

17. The method for estimating storage stability of a pharmaceutical composition comprising nicorandil, mannitol, and an alkali metal salt of an organic carboxylic acid according to claim 16, wherein
    the pharmaceutical composition is estimated to be stable at room temperature for 2 years, if the ratio of "a value of an X-ray diffraction peak of δ-form crystals of the mannitol at 9.8° ± 0.3° (2θ)"/"a value of an X-ray diffraction peak of nicorandil crystals at 10.6° ± 0.3° (2θ)" is 2.0 or less.

18. The method for estimating storage stability of a pharmaceutical composition comprising nicorandil, mannitol, and an alkali metal salt of an organic carboxylic acid according to claim 16, wherein
    the pharmaceutical composition is estimated to be stable at room temperature for two years, if the ratio of "an intensity of a peak of δ-form crystals of the mannitol at 1.01 THz ± 0.2 THz in a second derivative spectrum of an absorption spectrum obtained by terahertz transmission spectroscopy"/"an intensity of a peak of nicorandil crystals at 0.95 THz ± 0.2 THz in the second derivative spectrum of the absorption spectrum obtained by the terahertz transmission spectroscopy" is 1.5 or less.

[FIG. 1]

[FIG. 2]

[FIG. 3]

9.8° /10.6°

$R^2 = 0.9982$

[FIG. 4]

[FIG. 5]

[FIG. 6]

[FIG. 7]

[FIG. 8]

[FIG. 9]

[FIG. 10]

ABSORBANCE

FREQUENCY (T H z)

[FIG. 11]

ABSORBANCE

[FIG. 12]
SECOND-DERIVATIVE INTENSITY

[FIG. 13]
SECOND-DERIVATIVE INTENSITY

[FIG. 14]

1 . 0 1 T H z ／ 0 . 9 5 T H z INTENSITY RATIO

δ-FORM CRYSTALS OF D-MANNITOL/NICORANDIL MASS RATIO

[FIG. 15]

SECOND-DERIVATIVE INTENSITY

FREQUENCY（T H z）

[FIG. 16]

SECOND-DERIVATIVE INTENSITY

FREQUENCY ( T H z )

[FIG. 17]

SECOND-DERIVATIVE INTENSITY

FREQUENCY ( T H z )

[FIG. 18]

SECOND-DERIVATIVE INTENSITY

FREQUENCY ( T H z )

[FIG. 19]

SECOND-DERIVATIVE INTENSITY

FREQUENCY ( T H z )

<table>
<tr><td colspan="2" align="center">**INTERNATIONAL SEARCH REPORT**</td><td>International application No.<br>PCT/JP2012/066743</td></tr>
</table>

| A. | CLASSIFICATION OF SUBJECT MATTER |
|---|---|

*A61K31/455*(2006.01)i, *A61K9/19*(2006.01)i, *A61K47/10*(2006.01)i, *A61K47/12*
(2006.01)i, *A61P9/04*(2006.01)i, *A61P9/10*(2006.01)i

According to International Patent Classification (IPC) or to both national classification and IPC

| B. | FIELDS SEARCHED |
|---|---|

Minimum documentation searched (classification system followed by classification symbols)
A61K31/455, A61K9/19, A61K47/10, A61K47/12, A61P9/04, A61P9/10

Documentation searched other than minimum documentation to the extent that such documents are included in the fields searched
Jitsuyo Shinan Koho          1922–1996   Jitsuyo Shinan Toroku Koho   1996–2012
Kokai Jitsuyo Shinan Koho    1971–2012   Toroku Jitsuyo Shinan Koho   1994–2012

Electronic data base consulted during the international search (name of data base and, where practicable, search terms used)
CA/MEDLINE/EMBASE/BIOSIS(STN), JSTPlus/JMEDPlus/JST7580(JDreamII)

| C. | DOCUMENTS CONSIDERED TO BE RELEVANT |
|---|---|

| Category* | Citation of document, with indication, where appropriate, of the relevant passages | Relevant to claim No. |
|---|---|---|
| Y<br>A | JP 62-252722 A (Chugai Pharmaceutical Co., Ltd.),<br>04 November 1987 (04.11.1987),<br>examples<br>& US 4769381 A         & EP 236679 A1<br>& AU 8767562 A         & DE 3773017 G<br>& CA 1285485 C         & KR 9510150 B1 | 1-10,13-15<br>11,12,16-18 |
| Y<br>A | CN 101474161 A (Beijing Sihuan Kebao Pharmaceutical Co., Ltd.),<br>08 July 2009 (08.07.2009),<br>examples<br>(Family: none) | 1-10,13-15<br>11,12,16-18 |

☐ Further documents are listed in the continuation of Box C.       ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>03 September, 2012 (03.09.12) | Date of mailing of the international search report<br>18 September, 2012 (18.09.12) |
|---|---|
| Name and mailing address of the ISA/<br>Japanese Patent Office | Authorized officer |
| Facsimile No. | Telephone No. |

Form PCT/ISA/210 (second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP HEI713018 B
- JP HEI569088 B
- JP 2001187735 A